(19) Europäisches Patentamt
European Patent Office
Office européen des brevets

(11) Numéro de publication: **0 165 155**
**A1**

(12) # DEMANDE DE BREVET EUROPEEN

(21) Numéro de dépôt: **85401019.6**

(22) Date de dépôt: **23.05.85**

(51) Int. Cl.⁴: **C 07 D 491/04**
C 07 D 471/04, C 07 D 215/56
A 61 K 31/47, A 61 K 31/435
//(C07D491/04, 317:00, 221:00),
(C07D471/04, 221:00, 221:00)

(30) Priorité: **23.05.84 FR 8408036**

(43) Date de publication de la demande:
**18.12.85 Bulletin 85/51**

(84) Etats contractants désignés:
**BE CH DE GB IT LI LU NL SE**

(71) Demandeur: **S.A. PANMEDICA Société dite:**
**Zone Industrielle - Ilôt J**
**F-06516 Carros(FR)**

(72) Inventeur: **Laruelle, Claude**
**18 avenue Bellevue**
**F-06270 Villeneuve-Loubet(FR)**

(72) Inventeur: **Lepant, Marcel**
**7 rue Mellarede**
**F-06100 Nice(FR)**

(72) Inventeur: **Raynier, Bernard**
**"Les Glycines" 9 chemin du Malvan**
**F-06800 Cagnes Sur Mer(FR)**

(74) Mandataire: **Orès, Bernard et al,**
**Cabinet ORES 6, avenue de Messine**
**F-75008 Paris(FR)**

(54) **Nouveaux N-acyl dérivés d'amino acides et leurs esters, leur procédé de préparation et médicaments les contenant.**

(57) La présente invention est relative à de nouveaux N-acyl dérivés d'amino-acides.

Ces dérivés correspondent à la formule générale I ci-après:

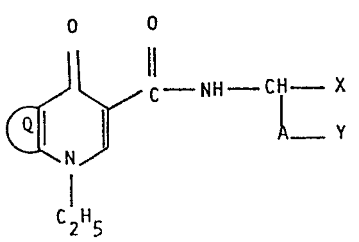

(I)

où A représente un radical hydrocarboné saturé, linéaire ou ramifié comprenant de 1 à 6 atomes de carbone, un cycle saturé à 6 atomes de carbone,
où X représente un radical -COOH, un radical -COOAlkyle (alkyle de C1 à C4), un atome d'hydrogène ou un groupe -CH3 quand Y est -COOH ou-COOAlkyle,
où Y représente un radical -COOH, un radical -COOAlkyle (de C1 à C4), un groupe -NH2, un groupe -SH, un groupe -SCH3, un groupe -NH-CH(=NH)-NH2, un groupe phényle substitué ou non, un groupe indol-3-yl, un groupe 5-hydroxy indol-3-yl, où Q représente un cycle aromatique éventuellement substitué, un hètérocyle comprenant 1 ou 2 atomes d'azote, éventuellement substitué.

Médicaments à action antiallergique.

EP 0 165 155 A1

La présente invention est relative à de nouveaux N-acyl dérivés d'amino acides et leurs esters, à leur procédé de préparation et aux médicaments contenant ces dérivés.

Les dérivés de 4-oxoquinoléines et de 4-oxonaphtyridines sont connus en tant que constituants de médicaments à puissante action antibactérienne ou antiallergique. A ce groupe appartiennent, par exemple, les antibactériens comme l'acide nalidixique, l'acide éthyl-1 fluoro-6 oxo-4 (pipérazine-1)-7 dihydro-1, naphtyridine-1,8 carboxylique-3 (enoxacine), l'acide pipémidique, l'acide oxolinique ou l'agent antiallergique comme le dérivé de naphtydrine décrit dans le Brevet US 4 303 661 par exemple.

La Demanderesse, en poursuivant la synthèse et l'étude de ce groupe extrêmement prometteur, a pu mettre au point une nouvelle série de dérivés possédant des propriétés pharmacologiques particulièrement intéressantes et notamment une action antiallergique, une action sur le système nerveux central et une action sur le système cardiovasculaire.

La présente invention a pour objet les dérivés 1-éthyl-4-pyridone-3-carboxyliques des amino acides ainsi que leurs esters, correspondant à la formule générale I ci-après :

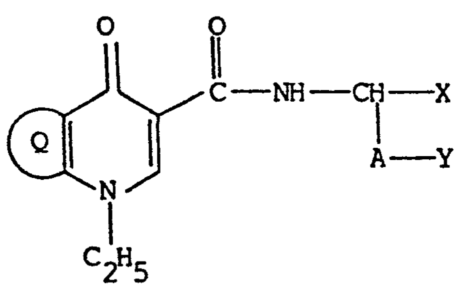

I

où A représente :

un radical hydrocarboné saturé, linéaire ou ramifié comprenant de 1 à 6 atomes de carbone,

un cycle saturé à 6 atomes de carbone.

où X représente :

un radical -COOH,

un radical -COOAlkyle (alkyle de C1 à C4),

un atome d'hydrogène ou un groupe $-CH_3$ quand Y est -COOH ou -COOAlkyle,

où Y représente :

un radical -COOH,

un radical -COOAlkyle (de C1 à C4),

un groupe -NH2,

un groupe -SH,

un groupe -SCH3,

un groupe -NH-CH(=NH)-NH2,

un groupe phényle substitué ou non,

un groupe indol-3yl

un groupe 5-Hydroxy indol-3 yl.

où Q représente :

un cycle aromatique éventuellement substitué

un hétérocycle comprenant 1 ou 2 atomes d'azote, éventuellement substitué.

La présente invention a également pour objet un procédé de fabrication de dérivés conformes à la présente invention, caractérisé en ce que l'on fait réagir un dérivé réactif de l'acide -1-éthyl-4-pyridone-3-carboxylique substitué avec un ester d'amino acide dans un solvant approprié, l'ester résultant pouvant être ensuite éventuellement débloqué par saponification classique, les réactions ayant lieu dans des solvants appropriés aux N-acylations.

Selon un mode de réalisation avantageux du procédé objet de la présente invention, les éventuelles fonctions réactives autres que la fonction NH2- de l'ester d'amino acide utilisé ont été bloquées.

Le déblocage de ces fonctions protégées est ensuite réalisé selon les techniques habituelles utilisées en synthèse peptidique.

Selon un autre mode de réalisation avantageux du procédé objet de la présente invention, la fonction amine de l'ester de l'amino acide utilisé est sous forme de son composé phosphorazo.

Outre les dispositions qui précèdent, l'invention comprend encore d'autres dispositions qui ressortiront de la description qui va suivre.

L'invention sera mieux comprise à l'aide du complément de description qui va suivre, qui se réfère à un compte-rendu pharmacologique et à des exemples de mise en oeuvre du procédé objet de la présente invention.

Il doit être bien entendu toutefois, que ces exemples de mise en oeuvre et les parties descriptives correspondantes sont donnés uniquement à titre d'illustration de l'objet de l'invention.

EXEMPLE 1-PREPARATION DE N-(1-ETHYL-1,4-DIHYDRO-6,7-METHYLENEDIOXY-4-OXOQUINOLEINE-3-CARBONYL)-3-AMINO BUTYRATE D'ETHYLE

A une suspension de 28,25 g (108 mM) d'acide -1-éthyl-1,4 dihydro-6,7 méthylène dioxy-4-oxoquinoléine-3 carboxylique dans 500 ml de diméthyl formamide on ajoute 140 mMoles de triéthylamine diluées dans du diméthyl formamide puis ajoute lentement à 0 °C 140 mM de chloroformiate d'éthyle. Après deux heures d'agitation à température ambiante, on ajoute 15,10 g (115 mM) d'amino -3- butyrate d'éthyle dilué dans 80 ml de DMF. On agite à température ordinaire pendant plusieurs jours jusqu'à fin de condensation.

On évapore ensuite sous vide, reprend au chloroforme et lave la couche organique successivement par la soude diluée, l'eau, l'acide chlorhydrique normal et enfin à l'eau. Après évaporation du solvant, on purifie par chromatographie sur silice dans un système chloroforme-acétone et l'on obtient le dérivé du titre avec un rendement de 50 % - pF 180 °C.

EXEMPLE 2 - ACIDE N-(1-ETHYL-1,4 DIHYDRO-6,7-METHYLENEDIOXY-4-OXOQUINOLEINE -3-CARBONYL)-3-AMINO BUTYRIQUE

On met en suspension 13,6 g (36,4 mM) de l'ester préparé selon l'exemple 1 dans 150 ml d'éthanol et 90 ml de soude normale. On agite à température ordinaire jusqu'à fin de saponification, soit environ 20 heures. On évapore ensuite l'éthanol, on reprend par l'eau, on traite au noir de charbon et on précipite par l'acide chlorhydrique dilué. Après filtration, lavage à l'eau et séchage, on obtient le dérivé du titre sous forme d'un produit cristallisé blanc de pF = 295 °C avec un rendement de 80 %. Sa chromatographie en couche mince sur plaque de silice dans le système chloroforme 25, acétone 7 présente un spot de Rf = 0,4 visible en U.V.

EXEMPLE 3 - N-(1-ETHYL-1,4-DIHYDRO-6,7-METHYLENEDIOXY-4-OXO QUINOLEINE-3-CARBONYL)-4-AMINOBUTYRATE DE TERT-BUTYLE

On dissout 12,7 g (80 mM) de l'ester tert-butylique de l'acide 4-aminobutyrique dans 500 ml de pyridine puis ajoute 22,36 g (80 mM) du chlorure de l'acide 1-éthyl-1,4-dihydro-6,7-méthylènedioxy-4-oxoquinoléine-3 carboxylique.

Après 8 heures de chauffage vers 100 °C, on laisse agiter 20 heures à température ordinaire, évapore sous vide à basse température puis reprend le résidu au chloroforme; après plusieurs lavages par HCl dilué, soude normale et eau, la couche organique est évaporée. Le produit est ensuite purifié par chromatographie sur silice dans un système chloroforme/ méthanol. On obtient ainsi le dérivé du titre sous forme de cristaux blancs de pF 192°C présentant en CCM sur plaque Kieselgel un seul spot de Rf = 0,55 (U.V.) dans le système solvant chloroforme 30/méthanol 2.

EXEMPLE 4 - ACIDE N-(1-ETHYL-1,4-DIHYDRO-6,7-METHYLENEDIOXY-4-OXOQUINOLEINE -3-CARBONYL)4-AMINO BUTYRIQUE

On met en suspension 10,5 g (26,2 moles) de l'ester préparé selon l'exemple 3, dans 20 ml d'acide acétique glacial puis ajoute à température ordinaire, 60 ml d'acide acétique préalablement saturé en acide chlorhydrique. On bouche hermétiquement et agite pendant 2 heures. On évapore ensuite partiellement l'excès de gaz chlorhydrique puis dilue à l'éther pour précipiter le produit brut. Après dissolution dans un excès de soude diluée, traitement au noir et reprécipitation par HCl dilué, on filtre et lave le précipité. On obtient ainsi le dérivé du titre avec un rendement de 82 % sous forme de cristaux blancs de pF = 230 °C. En C.C.M. sur plaque Kieselgel dans le système : n butanol 8/acide acétique 1/eau 1, le produit présente un spot de Rf = 0,65.

EXEMPLE 5 - N-(1-ETHYL-3,4-DIHYDRO-6,7-METHYLENEDIOXY-4-OXO QUINOLEINE-3-CARBONYL)-3-AMINO PROPIONATE DE BENZYLE

On met en suspension 18,55 g (71 mM) d'acide -1- éthyl-3,4-dihydro-6-7-méthylènedioxy-4-oxoquinoléine-3-carboxylique dans 300 ml de diméthyl formamide, ajoute 92 mM de triéthylamine dissous dans un peu de diméthyl formamide, puis coule à 0 °C 10 g (92 mM) de chloroformiate d'éthyle. Après une heure à température ordinaire on coule 80 mM de 3- amino propionate de benzyle en solution dans 100 ml de DMF et laisse agiter le mélange réactionnel 24 heures à température ambiante. Après évaporation du solvant à basse température, on reprend au chloroforme qu'on lave successivement par l'acide chlorhydrique dilué, la soude diluée puis par l'eau. Le solvant est évaporé et le produit est purifié par chromatographie sur colonne de silice avec un mélange chloroforme acétone.

On obtient 65 % du dérivé du titre sous forme de cristaux de pF 180 °C et présentant un seul spot en CCM sur plaque de silice dans le système chloroforme 30/méthanol 1 de Rf = 0,75.

EXEMPLE 6 - N-(1-ETHYL-3,4-DIHYDRO-6,7-METHYLENEDIOXY-4-OXOQUINOLEINE-3-CARBONYL)-3-AMINO PROPIONIQUE

On met en suspension 10,95 g (26 mM) de l'ester préparé à l'exemple 5 dans 150 ml d'éthanol, ajoute 65 ml de soude normale et laisse agiter à température ordinaire jusqu'à solubilisation totale. On traite au noir puis acidifie par l'acide chlorhydrique dilué, le précipité formé est filtré, lavé à l'eau et séché.on obtient ainsi le dérivé du titre avec un rendement de 98% sous forme de cristaux blancs de pF = 290 °C et présentant un seul spot en CCM sur silice dans le système n butanol 8/acide acétique 1/eau 1 de Rf=0,60.

EXEMPLE 7 - N-(1-ETHYL-1,4-DIHYDRO-6,7-METHYLENEDIOXY-4-OXOQUINOLEINE-3 CARBONYL)-6-AMINO HEXANOATE D'ETHYLE

On dissout 39,72 g (0,12 Mole) de 6-amino hexanoate d'éthyle dans 500 ml de pyridine puis ajoute à 0 °C 0,12 mole de chlorure de l'acide -1 éthyl-1,4-dihydro-6,7-méthylènedioxy-4 oxoquinoléine-3 carboxylique. On laisse revenir à température ambiante puis porte à 100 °C pendant 15 heures; après évaporation du solvant à basse température et reprise par l'acide chlorhydrique dilué, on extrait au chloroforme. La couche organique après lavage par l'acide chlorhydrique et la soude dilués puis par l'eau, est évaporée à sec. Le résidu est repris à l'éther puis recristallisé dans l'alcool isopropylique. On obtient ainsi le dérivé du titre sous forme de cristaux blancs de pF = 130 °C et présentant en C.C.M. sur plaque de silice dans le système toluène 10/formiate d'éthyle 10/acide formique 1 un seul spot de Rf 0,40.

EXEMPLE 8 - ACIDE N-(1-ETHYL-1,4-DIHYDRO-6,7-METHYLENEDIOXY-4-OXOQUINOLEINE-3 CARBONYL)-6-AMINO HEXANOIQUE

On met en suspension 12,15 g (30,2 mM) de l'ester obtenu à l'exemple 7 dans 60 ml d'éthanol puis ajoute 26 ml de soude normale. Après 15 heures d'agitation, la solution est complète, on ajoute 50 ml d'eau puis acidifie par l'acide chlorhydrique dilué. Le précipité formé est filtré, lavé, séché ; on obtient ainsi le dérivé du titre à l'état pur sous forme de cristaux blancs de pF = 210 °C présentant en C.C.M. sur plaque de silice un seul spot de Rf =0,25 dans le système toluène 10/formiate d'éthyle 10/acide formique 1.

EXEMPLE 9 - [L]N-(1-ETHYL-1,4-DIHYDRO-6,7-METHYLENEDIOXY-4-OXOQUINOLEINE-3 CARBONYL)-GLUTAMATE DE DIETHYLE

On dissout 23 G (0,13 M) de diéthyle glutamate dans 750 ml de pyridine puis ajoute à 0 °C, 0,13 Mole du chlorure de l'acide-1-éthyl-1,4-dihydro-6,7-méthylènedioxy-4-oxo quinoléine-3-carboxylique. On chauffe à 100 °C pendant 15 heures, filtre à chaud un léger insoluble puis évapore la solution. On reprend le résidu par l'acide chlorhydrique dilué, glacé et extrait par le chloroforme. La solution organique est lavée de façon habituelle, puis après évaporation est chromatographiée sur colonne de silice. On obtient ainsi le dérivé du titre sous forme de cristaux blancs de pF = 159 °C et présentant en C.C.M. sur plaque de silice dans le système chloroforme 25/acétone 5 un seul spot de Rf = 0,45.

EXEMPLE 10 - ACIDE (L) N-[1-ETHYL-1,4-DIHYDRO-6,7-METHYLENEDIOXY-4-OXOQUINOLEINE-3-CARBONYL]-GLUTAMIQUE

On met en suspension 18,50 g (32, mM) d'ester décrit à l'exemple 9 dans 100 ml d'éthanol en 98 ml de soude normale. On agite à température ordinaire jusqu'à solubilisation totale puis dilue à l'eau, traite au noir et précipite par l'acide chlorhydrique dilué. Après filtration, lavage à l'eau et séchage, on obtient le dérivé du titre sous forme de cristaux blancs de pF = 237 °C et présentant un seul spot en C.C.M. sur plaque de silice dans le système butanol 8/acide acétique 1/eau 1, de Rf = 0,45.

EXEMPLE 11 - [L] N-[-1 ETHYL-1,4-DIHYDRO-6,7-METHYLENEDIOXY-4-OXOQUINOLEINE-3-CARBONYL]-ASPARTATE DE DIETHYLE

On dissout 115 mMoles de (L) aspartate de diéthyle dans 400 ml de pyridine puis ajoute lentement vers + 5 °C 32,15 g (115 mM) du chlorure de l'acide -1-éthyl-1,4-dihydro-6,7-méthylènedioxy-4-oxoquinoléine-3-carboxylique. On chauffe alors à 100 °C pendant 5 heures, puis laisse agiter à température ordinaire pendant 16 heures. On évapore ensuite sous pression réduite et reprend le résidu par 400 ml d'acide chlorhydrique normale. Le précipité formé est filtré, lavé à l'eau et séché.

On purifie ensuite par chromatographie sur colonne de silice dans un système chloroforme/acétone pour obtenir le dérivé du titre sous forme de cristaux blancs de pF = 148 °C présentant un seul spot en C.C.M. sur plaque de silice dans le système chloroforme 20/acétone 5 de Rf = 0,45.

EXEMPLE 12-ACIDE (L)N-[1-ETHYL-1,4-DIHYDRO-6,7-METHYLENEDIOXY-4-OXOQUINOLEINE -3-CARBONYL]-ASPARTIQUE

On met en suspension 21,6 g (50 mM) du diester décrit à l'exemple 11 dans 100 ml d'éthanol et 150 ml de soude normale. On laisse agir 24 heures à température ordinaire, traite au noir puis acidifie par l'acide chlorhydrique dilué. Le précipité formé est filtré, lavé, séché. On obtient ainsi le dérivé du titre avec un rendement de 90 % sous forme de cristaux blancs de pF=221 °C présentant en C.C.M. sur plaque de silice dans le système n Butanol 8/acide acétique 1/eau 1 un seul spot de Rf = 0,30.

EXEMPLE 13 - N-[1-ETHYL-1,4-DIHYDRO-6,7-METHYLENEDIOXY-4-OXO-QUINOLEINE-3-CARBONYL]-4-AMINOMETHYL CYCLOHEXANE CARBOXYLATE D'ETHYLE

A une suspension de 29,5 g (113 mM) d'acide -1-éthyl-1,4-dihydro-6,7-méthylène dioxy-4-oxo-quinoléine-3 carboxylique dans 450 ml de diméthyl formamide, on ajoute 14,2 g (140 mM) de triéthylamine puis 14,8 g (137 mM) de chloroformiate d'éthyle. Après 2 heures d'agitation à température ambiante, on ajoute 113 mMoles de 4-aminométhyl cyclohexane carboxylate d'éthyle. On agite la suspension à température ambiante pendant plusieurs jours jusqu'à fin de réaction. On évapore sous vide la majeure partie du solvant puis reprend par le chloroforme qu'on lave de façon habituelle. Après évaporation du solvant, on purifie le produit brut par chromatographie sur silice par un mélange chloroforme acétone. On obtient ainsi le dérivé du titre à l'état pur (pF = 214 °C). T.L.C. sur silice dans le système chloroforme 30/isopropranol 1,5 un seul spot de Rf = 0,45.

EXEMPLE 14 - ACIDE N-[1-ETHYL-1,4-DIHYDRO-6,7-METHYLENEDIOXY-4-OXOQUINOLEINE -3 CARBONYL]-4-AMINOMETHYL CYCLOHEXANE CARBOXYLIQUE

On met en suspension 3,43 g (8 mM) de l'ester précédent dans 35 ml d'éthanol, 20 ml de soude normale et 20 ml d'eau. Après 15 heures d'agitation à température ambiante, on acidifie par l'acide chlorhydrique dilué, filtre et lave le précipité. On obtient après séchage le dérivé du titre à l'état pur avec un rendement de 72 % (pF = 301 °C) T.L.C. sur silice dans le système chloroforme 30/isopropanol 1,5 un seul spot de Rf = 0,15.

EXEMPLE 15 - N-[1-ETHYL-1,4-DIHYDRO-7-METHYL-4-OXO-1,8-NAPHTYRIDINE-3-CARBONYL]-3 AMINO PROPIONATE D'ETHYLE

On ajoute 6,90 g (50 mM) de trichlorure de phosphore à une solution de chlorhydrate de 3-amino propionate d'éthyle (15,3 g ; 0,1 M) dans 400 ml de pyridine. Après 2 heures d'agitation à température ambiante, on ajoute 23,1 g (0,1 M) d'acide 1-éthyl-1,4-dihydro-7-méthyl-4-oxo-1,8-naphtyridine-3 carboxylique et chauffe à 105/115 °C pendant 16 heures. Après refroidissement on évapore le solvant sous pression réduite, puis reprend le résidu par l'acide chlorhydrique normal. La solution aqueuse est extraite au chloroforme,celui-ci est lavé à l'acide chlorhydrique puis à la soude dilués et enfin à l'eau. Après évaporation du solvant, le résidu est repris à l'éther, puis à l'éthanol chaud. Après filtration et séchage, on obtient le dérivé du titre sous forme de cristaux blancs de pF 178 °C présentant un seul spot en T.L.C. sur plaque de silice dans le système toluène 10/formiate d'éthyle 10/acide formique 1 de Rf = 0,50.

EXEMPLE 16 - ACIDE N-[1-ETHYL-1,4-DIHYDRO-7-METHYL-4-OXO-1,8-NAPHTYRIDINE-3-CARBONYL]-3 AMINO PROPIONIQUE

- Par hydrolyse de l'ester éthylique :

On met en suspension 0,1 Mole de l'ester décrit à l'exemple ci-dessus dans 500 ml d'éthanol et 150 ml de soude normale et agite à température ordinaire pendant 15 heures. On évapore l'éthanol sous pression réduite, dilue à l'eau puis précipite par acidification. Après filtration, lavages à l'eau et séchage, on obtient le dérivé du titre sous forme de cristaux blancs de pF = 252 °C avec un rendement de 70 % La T.L.C. du dérivé sur silice dans le système toluène 10/formiate d'éthyle 10/acide formique 1 présente un seul spot de Rf = 0,30.

- Par hydrolyse de l'ester benzylique :

N-[1-éthyl-1,4-dihydro-7-méthyl-4-oxo-1,8-naphtyridine-3-carbonyl]-3-amino propionate de benzyle.

On dissout 45,7 g (0,13 M) de paratoluène sulfonate de 3-amino propionate de benzyle dans 450 ml de pyridine puis ajoute vers + 5 °C, 9 g de trichlorure de phosphore (65 mM).

Après 2 heures à température ordinaire, on ajoute 30,2 g (0,13 M) d'acide 1-éthyl-1,4-dihydro-7-méthyl-4-oxo-1,8-naphtyridine-3-carboxylique et chauffe à 105° - 110 °C pendant 15 heures. Après traitement identique à celui de l'ester éthylique précédant, on obtient le dérivé du titre de pF 157 °C avec un rendement de 90 %.

- Hydrolyse de l'ester benzylique :

On met en suspension 0,1 Mole de l'ester précédemment décrit dans 500 ml d'éthanol et 150 ml de soude normale. Après 15 heures d'agitation à température ambiante, l'éthanol est évaporé sous pression réduite et après dilution à l'eau, on acidifie pour précipiter la forme acide, identique en tous points au produit obtenu par hydrolyse de l'ester éthylique.

EXEMPLE 17 - N-[1-ETHYL-1,4-DIHYDRO-7-METHYL-4-OXO-1,8-NAPHTYRIDINE-8 CARBONYL]-3 AMINO BUTYRATE D'ETHYLE

On dissout 13,1 g (0,1 M) de 3-amino butyrate d'éthyle dans 400 ml de pyridine, puis ajoute vers 10°, 6,9 g (50 mM) de trichlorure de phosphore. Après 2 heures d'agitation à l'ambiance, on additionne 23,2 g d'acide 1-éthyl -1,4-dihydro-7-méthyl-4-oxo-1,8-naphtyridine-3 carboxylique. On maintient 15 heures à 100 - 105 °C, évapore le solvant sous pression réduite puis par extraction au chloroforme et lavage de façon habituelle, on obtient le dérivé du titre à l'état pur après chromatographie sur silice par un mélange chloroforme/acétone - pF = 107 °C. T.L.C. (silice : chloroforme 30/méthanol 2 - Rf = 0,60).

EXEMPLE 18 - ACIDE N-[1-ETHYL-1,4-DIHYDRO-7-METHYL-4-OXO-1,8-NAPHTYRIDINE-3-CARBONYL]-3 AMINO BUTYRIQUE

On met en suspension 4,7 g (13,6 mM) de l'ester précédemment obtenu en suspension dans 60 ml d'éthanol et 27 ml de soude normale. On agite pendant 16 heures à température ordinaire, évapore l'éthanol, dilue à l'eau, puis précipite à froid par l'acide chlorhydrique dilué. Après filtration, lavage et séchage, on obtient le dérivé du titre à l'état pur sous forme de cristaux blancs de pF = 254 °C avec un rendement de 93 % (T.L.C. sur silice dans n butanol 80/eau 10/acide acétique 1, un seul spot de Rf = 0,80).

EXEMPLE 19 - N-[1-ETHYL-1,4-DIHYDRO-7-METHYL-4-OXO-1,8-NAPHTYRIDINE-3-CARBONYL]-4 AMINO BUTYRATE DE TER-BUTYLE

On dissout 13,5 g (85 mM) de 4-amino butyrate de tert-butyle dans 250 ml de pyridine puis coule 5,5 g (40 mM) de trichlorure de phosphore, dilué dans un peu de pyridine. On agite 2 heures à température ambiante puis ajoute 18,2 g d'acide -1-éthyl-1,4-dihydro-7-méthyl-4-oxo-1,8-naphtyridine-3-carboxylique. Après 7 heures de chauffage à 105/115 °C, on évapore le solvant sous pression réduite et reprend au chloroforme. Après lavage de l'extraction chloroformique de façon habituelle, on purifie le résidu brut d'évaporation du solvant par chromatographie sur colonne de silice par le mélange chloroforme/méthanol. On obtient le dérivé du titre sous forme de cristaux blancs de pF = 119 °C (T.L.C. sur silice dans le mélange chloroforme 20/méthanol 0,4 un seul spot de Rf = 0,60).

EXEMPLE 20 - ACIDE N-[1-ETHYL-1,4-DIHYDRO-7-METHYL-4-OXO-1,8-NAPHTYRIDINE-3-CARBONYL]-4-AMINO BUTYRIQUE

On met en suspension 6 g (16 mM) de l'ester préparé à l'exemple précédent dans 40 ml d'acide acétique glacial saturé en gaz chlorhydrique. Après 2 heures d'agitation, on évapore sous pression réduite et reprend à l'éther. Le produit est purifié par dissolution en milieu alcalin, traitement au noir et reprécipitation par l'acide chlorhydrique dilué. Après filtration, lavage, séchage, on obtient le dérivé du titre avec un rendement de 90 %.
(pF = 178 °C). T.L.C. sur silice dans le système toluène 10/formiate d'éthyle 10/acide formique 1, un seul spot Rf = 0,40.

EXEMPLE 21 - N-[1-ETHYL-1,4-DIHYDRO-7-METHYL-4-OXO-1,8-NAPHTYRIDINE-3-CARBONYL]-6-AMINO HEXANOATE D'ETHYLE

On dissout 39,8 g (0,12 M) de 6-amino hexanoate d'éthyle dans 600 ml de pyridine puis coule 8,3 g (60 mM) de trichlorure de phosphore. Après 2 heures d'agitation à température ambiante, on ajoute 27,9 g (0,12 M) d'acide -1-éthyl-1,4-dihydro-7-méthyl-4-oxo-1,8-naphtyridine-3-carboxylique puis porte à 100 °C pendant 16 heures. Après évaporation sous pression réduite et reprise à l'acide chlorhydrique dilué, on extrait au chloroforme. L'extraction organique est lavée de façon habituelle.

Après évaporation à sec sous pression réduite, le résidu est recristallisé dans l'alcool isopropylique. On obtient ainsi le dérivé du titre de pF = 113 °C. T.L.C. sur silice dans le système toluène 10/formiate d'éthyle 10/ acide formique 1, un seul spot de Rf 0,50.

EXEMPLE 22 - ACIDE N-[1-ETHYL-1,4-DIHYDRO-7-METHYL-4-OXO-1,8-NAPHTYRIDINE-3-CARBONYL]-6-AMINO CAPROIQUE

On met en suspension 19,5 g (52 mM) de l'ester préparé à l'exemple précédent, dans 90 ml d'éthanol et 50 ml de soude 2N. Après 18 heures d'agitation, on dilue à l'eau, traite au noir et acidifie par l'acide chlorhydrique concentré. Le précipité, lavé, séché, est le dérivé du titre de pF = 163 °C. T.L.C. sur silice dans le système toluène 10/formiate d'éthyle 10/ acide formique 1 : un seul spot de Rf = 0,40.

EXEMPLE 23 - N-[1-ETHYL-1,4-DIHYDRO-7-METHYL-4-OXO-1,8-NAPHTYRIDINE-3-CARBONYL]-4-AMINOMETHYL CYCLOHEXANE CARBOXYLATE D'ETHYLE

On dissout 16,6 g (75 mM) de -4-amino méthyl cyclohexane carboxylate d'éthyle dans 300 ml de pyridine, ajoute 5,2 g (37 mM) de trichlorure de phosphore, puis après 2 heures à température ordinaire, ajoute 17,4 g (75 mM) d'acide -1-éthyl-1,4-dihydro-7-méthyl-4-oxo-1,8-naphtyridine-3 carboxylique. Après 15 heures à 115°/120 °C, on évapore le solvant, reprend par l'acide chlorhydrique normal et extrait au chloroforme, puis après le traitement habituel, le produit brut est recristallisé dans l'isopropanol.
On obtient ainsi le dérivé du titre avec un rendement de 50 % sous forme de cristaux blancs de pF = 165 °C. T.L.C. sur silice dans le système chloroforme 60/acétone 10 = un seul spot de Rf = 0,55.

EXEMPLE 24 - ACIDE-N-[1-ETHYL-1,4-DIHYDRO-7-METHYL-4-OXO-1,8-NAPHTYRIDINE-3-CARBONYL]-4-AMINOMETHYL CYCLOHEXANE CARBOXYLIQUE

On met en suspension 12 g (30 mM) de l'ester obtenu à l'exemple précédent dans 120 ml d'éthanol et 60 ml de soude normale. Après 18 heures d'agitation à température ambiante, on évapore l'éthanol, dilue à l'eau et précipite la forme acide par l'acide chlorhydrique dilué. Après filtration, lavage, séchage, on obtient le dérivé du titre pur sous forme de cristaux blancs de pF = 259 °C avec un rendement de 92 %. T.L.C. sur silice dans le système chloroforme 60/acétone 10 = un seul spot de Rf = 0,40.

EXEMPLE 25 - (L)-N-[1-ETHYL-1,4-DIHYDRO-7-METHYL-4-OXO-1,8-NAPHTYRIDINE-3-CARBONYL]-ASPARTATE DE DIMETHYLE

On dissout 8,50 g (58 mM) de (L)aspartate de diméthyle dans 220 ml de pyridine, ajoute 4 g (29 mM) de trichlorure de phosphore, puis après 2 heures d'agitation à l'ambiance, ajoute 58 mM d'acide-1-éthyl-1,4-dihydro-7méthyl-4-oxo-1,8-naphtyridine-3-carboxylique. On porte à reflux 8 heures puis après évaporation du solvant et reprise par 400 ml d'acide chlorhydrique, extrait par le chloroforme. Après lavages de l'extraction organique selon les techniques habituelles et évaporation du chloroforme, le résidu brut est purifié par chromatographie sur silice par un mélange chloroforme acétone. On obtient ainsi le dérivé du titre sous forme de cristaux blancs de pF =135 °C. T.L.C. sur silice dans chloroforme 80/acétone 10 ; un seul spot de Rf = 0,55.

EXEMPLE 26 - (L)-N-[1-ETHYL-1,4-DIHYDRO-7-METHYL-4-OXO-1,8-NAPHTYRIDINE-3-CARBONYL]-ASPARTATE DE DIETHYLE

On opère comme dans l'exemple précédent en utilisant 58 mM de -L-aspartate de diéthyle pour obtenir le dérivé du titre avec un rendement de 77 %, un pF de 103 °C. T.L.C. sur silice dans le système benzène 8/acétone 2 ; un seul spot de Rf = 0,70.

EXEMPLE 27 - ACIDE (L)-N-[1-ETHYL-1,4-DIHYDRO-7-METHYL-4-OXO-1,8-NAPHTYRIDINE-3-CARBONYL]-ASPARTIQUE

On met en suspension 24,2 g (60 mM) d'ester précédent dans 125 ml d'éthanol et 180 ml de soude normale et agite à température ordinaire pendant 18 heures. On acidifie ensuite par l'acide chlorhydrique dilué, filtre, lave et sèche le précipité. On obtient ainsi le dérivé du titre sous forme de cristaux blancs de pF = 207 °C avec un rendement de 70 %. T.L.C. sur silice dans le système n butanol 8/eau 1/acide acétique 1 ; un seul spot de Rf=0,40.

EXEMPLE 28 - (L)-N-[1-ETHYL-1,4-DIHYDRO-7-METHYL-4-OXO-1,8-NAPHTYRIDINE-3-CARBONYL]-GLUTAMATE DE DIETHYLE

On dissout 4,8 g (20 mM) de chlorhydrate de L-diéthyle glutamate dans 40 ml de pyridine puis ajoute 1,4 g (10 mM) de trichlorure de phosphore dilué dans 15 ml de pyridine. On agite une heure à température ordinaire puis ajoute 4,65 g (20 mM) d'acide -1-éthyl-1,4-dihydro-7-méthyl-4-oxo-1,8-naphtyridine-3-carboxylique.

Après 7 heures de reflux, on concentre sous pression réduite, reprend au chloroforme, qu'on lave de façon habituelle. Après évaporation à sec, on obtient le dérivé du titre à l'état pur de pF = 103 °C.

T.L.C. sur silice dans le système benzène 8/méthanol 2 ; un seul spot de Rf = 0,80.

EXEMPLE 29 - ACIDE (L)-N-[1-ETHYL-1,4-DIHYDRO-7-METHYL-4-OXO-1,8-NAPHTYRIDINE -3-CARBONYL]-GLUTAMIQUE

On met en suspension 4,2 g (10 mM) d'ester précédent dans 20 ml d'éthanol et 30 ml de soude normale. Après quelques heures d'agitation à température ordinaire, on acidifie par l'acide chlorhydrique dilué. On filtre le précipité, lave à l'eau puis reprend par un mélange éthanol/éther. L'insoluble après filtration et séchage représente le dérivé du titre à l'état pur pF = 220° C avec un rendement de 70 %. T.L.C. sur silice dans le système : n butanol 8/acide acétique 1/eau 1 ; un seul spot de Rf = 0,60.

COMPTE RENDU PHARMACOLOGIQUE

L'ensemble des produits selon l'invention possède des propriétés anti-allergiques. Ainsi, injectés à des doses de 12,5 et 25 mg/kg, ils présentent des propriétés protectrices vis-à-vis de la réaction anaphylactique déclenchée par injection intraveineuse d'ovalbumine à des rats sensibilisés. Injectés à des doses comprises entre 5 et 50 mg/kg, les dérivés selon l'invention, induisent chez des rats spontanément hypertendus un effet anti-hypertenseur comparable à celui de l'alpha méthyldopa. L'activité cardiaque inotrope positive mesurée sur le coeur isolé de cobaye est supérieure à celle de l'amrinone. Enfin, les activités bronchodilatatrices des dérivés selon l'invention testés sur l'organe isolé sont comparables à celle de l'aminophylline. D'autre part, les toxicités des dérivés de la présente invention mesurées selon la technique de la DL 50/souris sont faibles et en général supérieures à 2000 mg/kg per os.

Les produits selon l'invention peuvent être administrés à l'animal de façon conventionnelle seuls ou en combinaison avec d'autres agents thérapeutiques. Le dosage de la forme administrée varie selon des caractéristiques pharmacodynamiques du produit considéré et le mode d'administration.

Ce dosage varie également en fonction de l'âge du patient, de la nature et de l'étendue des symptômes mais aussi en fontion des effets désirés.

Habituellement, une dose journalière de produit actif peut être comprise entre 5 et 20 milligrammes par kilo de poids corporel.

Le principe actif peut être administré oralement sous des formes galéniques solides telles que comprimés, capsules, poudres, sous des formes liquides, telles que sirop ou suspension ; il peut être également administré par voie parentérale en solution dans un solvant approprié stérile.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## REVENDICATIONS

1 - Dérivés 1-éthyl-4-pyridone-3-carboxyliques des amino-acides ainsi que leurs esters, correspondant à la formule générale I ci-après :

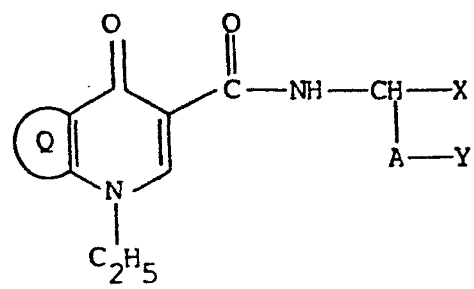

I

où A représente :

un radical hydrocarboné saturé, linéaire ou ramifié comprenant de 1 à 6 atomes de carbone,

un cycle saturé à 6 atomes de carbone.

où X représente :

un radical -COOH,

un radical -COOAlkyle (alkyle de C1 à C4),

un atome d'hydrogène ou un groupe -CH3 quand Y est -COOH ou -COOAlkyle,

où Y représente :

un radical -COOH,

un radical -COOAlkyle (de C1 à C4),

un groupe -NH2,

un groupe -SH,

un groupe -SCH3,

un groupe -NH-CH(=NH)-NH2,

un groupe phényle substitué ou non,

un groupe indol-3yl

un groupe 5-Hydroxy indol-3 yl.

où Q représente :

un cycle aromatique éventuellement substitué

un hétérocycle comprenant 1 ou 2 atomes d'azote, éventuellement substitué.

2- Sels d'addition des composés selon la revendication 1 avec les acides pharmaceutiquement acceptables.

3- Sels d'addition des composés selon la revendication 1 avec les bases pharmaceutiquement acceptables.

4- Les dérivés selon les revendications 1 à 3 où Q détermine avec la pyridone, le dérivé -1-éthyl-1,4-dihydro-6,7-méthylènedioxy-4-oxo-quinoléine-3-yl.

5- Les dérivés selon les revendications 1 à 3 où Q détermine avec la pyridone, le dérivé -1-éthyl-1,4-dihydro-7-méthyl-4-oxo-1,8-naphtyridine-3-yl.

6- Dérivé selon la revendication 1, caractérisé en ce qu'il est constitué par l'acide N-(1-éthyl-1,4-dihydro-6,7-méthylènedioxy-4-oxoquinoléine-3-carbonyl)-4-amino-butyrique.

7- Dérivé selon la revendication 1, caractérisé en ce qu'il est constitué par le -(L)N-(1-éthyl-1,4-dihydro-6,7-méthylènedioxy-4-oxoquinoléine-3-carbonyl)-glutamate de diéthyle.

8- Dérivé selon la revendication 1, caractérisé en ce qu'il est constitué par le (L)N-(1-éthyl-1,4-dihydro-6,7-méthylènedioxy-4-oxoquinoléine-3-carbonyl)-aspartate de diéthyle.

9- Dérivé selon la revendication 1, caractérisé en ce qu'il est constitué par le (L)N-(1-éthyl-1,4-dihydro-7-méthyl-1,8-naphtyridine-3-carbonyl)-aspartate de diéthyle.

10- Dérivé selon la revendication 1, caractérisé en ce qu'il est constitué par le (L)N(1-éthyl-1,4-dihydro-7-méthyl-4-oxo-1,8-naphtyridine-3-carbonyl)-glutamate de diéthyle.

11- Procédé de préparation des dérivés selon l'une quelconque des revendications 1 à 10, caractérisé en ce que l'on fait réagir un dérivé réactif de l'acide -1-éthyl-4-pyridone-3-carboxylique substitué avec un ester d'amino-acide dans un solvant approprié, l'ester résultant pouvant être ensuite éventuellement débloqué par saponification classique, les réactions ayant lieu dans des solvants appropriés aux N-acylations.

12- Procédé selon la revendication 11, caractérisé en ce que les éventuelles fonctions réactives autres que la fonction NH2- de l'ester d'amino-acide utilisé ont été bloquées.

13- Médicaments caractérisés en ce qu'ils contiennent au moins un composé selon les revendications 1 à 10, seul ou en association avec un ou plusieurs principes actifs et/ou adjuvants pharmaceutiquement compatibles.

Office européen des brevets

# RAPPORT DE RECHERCHE EUROPEENNE

0165155
Numéro de la demande
EP 85 40 1019

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 590 036 (G.Y. LESHER) * Revendication 1; colonne 10, lignes 29-51; colonne 52, exemples 136,137 * | 1,13 | C 07 D 491/04<br>C 07 D 471/04<br>C 07 D 215/56<br>A 61 K 31/47<br>A 61 K 31/435//<br>(C 07 D 491/04<br>C 07 D 317:00<br>C 07 D 221:00 )<br>(C 07 D 471/04<br>C 07 D 221:00<br>C 07 D 221:00 ) |
| A | US-A-3 524 858 (WARNER-LAMBERT) * Revendication 1; colonne 2, lignes 3-18 * | 1,13 | |
| A | EP-A-0 070 767 (ROUSSEL-UCLAF) * Revendication 1; page 5, lignes 21-27 * | 1,13 | |
| | | | **DOMAINES TECHNIQUES RECHERCHES (Int. Cl.4)** |
| | | | C 07 D 491/00<br>C 07 D 471/00<br>C 07 D 215/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 28-08-1985 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant